Europäisches Patentamt

European Patent Office

Office européen des brevets

(11) Publication number: **0 161 868 B1**

(12) **EUROPEAN PATENT SPECIFICATION**

(45) Date of publication of patent specification: **04.11.92**

(51) Int. Cl.5: **G01N 33/53**, G01N 33/543, G01N 33/545

(21) Application number: **85303100.3**

(22) Date of filing: **01.05.85**

(54) Fluorometric assay of IgG4.

(30) Priority: **11.05.84 US 609267**

(43) Date of publication of application:
**21.11.85 Bulletin 85/47**

(45) Publication of the grant of the patent:
**04.11.92 Bulletin 92/45**

(84) Designated Contracting States:
**DE**

(56) References cited:
**EP-A- 0 088 974**
**US-A- 4 410 634**
**US-A- 4 444 879**

**ANNALS OF ALLERGY, vol. 46, March 1981, US; N.-K.V.CHEUNG et al., pp. 132-136**

**ANALYTICAL BIOCHEMISTRY, vol. 117, 1981, Academic Press Inc., New York, NY (US); P.K.GOLDSMITH, pp. 53-60**

**HYBRIDOMA, vol. 1, no. 3, March 1982, Mary Ann Liebert Inc., Chicago, IL (US); T.A.SPRINGER et al., pp. 257-273**

(73) Proprietor: **BioWhittaker, Inc.**
**8830 Biggs Ford Road**
**Walkersville, Maryland 21793-0127(US)**

(72) Inventor: **Halpern, George M.**
**9 Hillbrook Drive**
**Portola Valley, CA 94025(US)**
Inventor: **Scott, John, R.**
**521 Del Medio, no. 103**
**Mountain View, CA 94043(US)**
Inventor: **Yuh-Geng, Tsay**
**1933 Cape Horn Drive**
**San Jose, CA 95133(US)**
Inventor: **Sun, Jane V.**
**13005 La Cresta Drive**
**Los Altos Hills, CA 94022(US)**

(74) Representative: **Armitage, Ian Michael et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

ENZYME-IMMUNOASSAY, E.T.Maggio (ed.), CRC Press, Boca Raton, FL (US), 1980; pp. 181-193

KLIN. WOCHENSCHRIFT, vol. 58, 1980; R.URBANEK et al., pp. 1257-1260

KLIN. WOCHENSCHRIFT, vol. 57, 1979; FOR-STER et al., pp. 421-422

## Description

This invention relates to methods and reagents for assaying blood serum of patients demonstrating allergic symptomotology to identify the source of the allergy and determine the level of the respective allergen specific IgG₄ in the serum. In particular, this invention relates to diagnostic methods and reagents therefor which provide increased specificity and accuracy, the results of which can be reliably used to monitor IgG₄ levels in patent serum before and during allergic desensitization.

Radiometric and fluorometric methods for identifying and measuring antigen levels in patient serum are commercially available and are known as the RAST test, for example. U.S. patents RE-29,474; 3,555,143; 3,648,346; 3,720,760, and 3,966,898 relate to these methods and reagents therefor. Enzymatic immunological methods for identifying and quantifying antigens and Haptens in liquids are widely used and are known as the ELISA and EIA, for example. Basic technology for enzymatic assays and reagents therefor is disclosed in U.S. patents RE-29,169 and 3,839,153, for example.

A review of the current state of the art with regard to immunoassays for the detection of proteins in solutions is provided by R. Rose et al, Manual of Clinical Immunology, 2nd ed. American Society for Microbiology, Washington, pp 327-429, 775-849 (1980) and by A. Voller et al, Immunoassays for the 80's, University Park Press, Baltimore (1981), and the publications cited therein. The chapter therein by T. A. E. Platts-Mills et al, "Radioimmunoassays in Allergy", pp 289-311, and the publications cited therein provide a comprehensive review of the field of this invention.

A variety of sensitive immunoassays for specific human IgG₄ levels are described in Halpern, G.M. Clinical Reviews in Allergy, 1;207-236 (June, 1983) and numerous literature reviews included therein. Various methods of detecting specific IgG₄ antibodies have been described. The radioallergosorbent test (RAST) are described in Bruynzeel, P.L.B. et al "IgE and IgG₄ antibodies in specific human allergies." Int. Arch. Allergy. Appl. Immun. 58:344-350 (1979); Iskander, R. et al "IgG₄ antibodies in Egyptian patients with Schistosomiasis." Int. Arch. Allergy Appl. Immunol. 66:200-207 (1981); Van der Giessen, M. et al "Subclass typing of IgG antibodies formed by grass pollen-allergic patients during immunotherapy." Int. Arch. Allergy Appl. Immmun. 50:625-640 (1976). Polystyrene tube radioimmunoassay (RIA) are described in Kramps, J.A. et al "Occupational asthma due to inhalation of chloramine-T. II. Demonstration of specific IgE antibodies." Int. Arch. Allergy Appl. Immunol. 64:428-438 (1981). Enzyme-linked immunosorbent assay (ELISA) are described in Urbanek, R. et al "Immunological studies on bee-keepers: Specific IgG and subclass typing IgG against bee venom and bee venom components." Klin. Wochenschr. 58:1257-1260 (1980); Etievant, M. et al "Immuno-enzymatic study of IgG subclasses specific for allergen in house dust immediate hypersensitivity." Ann. Allergy 43:169-173 (1979); Weits, J. et al "Class and subclass-specific antibody response to hemocyanin in nonmalignant paraproteinemia." J. Lab. Clin. Med. 94:458-466 (1979). Quantitative immunofluorescence defined antigen substrate spheres system (DASS) are described in Van der Giessen, M. et al "Subclass typing of IgG antibodies formed by grass pollen-allergic patients during immunotherapy." Int. Arch. Allergy Appl. Immun. 50:625-640 (1976) . Red cell linked antigen-antiglobulin reaction (RCLAAR) are described in Devey, M.E. et al "The IgG subclasses of antibodies to castor bean allergen in patients with allergic asthma: detection of a high incidence of antibodies of the IgG₄ subclass. Clin. Allergy 5:353-361 (1975). microtiter solid phase radioimmunoassay (MSPRIA) are described in Cheung, N-KV. et al "Honeybee venom specific immunoglobulin G₄ in honeybee allergic patients and beekeepers." Ann. Allergy (1983). Histamine release is described in Assem, E.S. et al "Cytophilic antibodies in bronchopulmonary aspergilloma and cryptogenic pulmonary eosinophilia." Clin. Exp. Immunol. 26:67-77 (1976); Vijay, H.M. et al "Possible role of IgG₄ in discordant correlations between intracutaneous skin tests and RAST." Int. Arch. Allergy Appl. Immunol. 56:517-522 (1978).

RAST, polystyrene tube RIA, and MSPRIA are all radioimmunoassays, in which antigen-specific antibodies in serum samples bind to solid-phase-bound antigens and can thus be detected by radiolabeled subclass-specific reagents. In the RAST method, antigens are chemically coupled to cellulose or Sepharose 4B using cyanogen bromide. Serum samples are allowed to incubate at room temperature with these antigen-coupled cellulose disks or Sepharose, usually for 24 hours. The disks or Sepharose are then washed extensively and developed with radiolabeled (¹²⁵I) human-IgG₄-specific affinity column purified antibodies. The results are usually expressed as percentages of added radioactivity.

Procedures for binding proteins to insoluble supports have been primarily described. US 4410634 describes a method in which specific immunoreactive haptens are covalently bound to a macromolecular carrier and the resulting hapten-carrier conjugate is adsorbed to the surface of a solid phase. Antibodies have also been covalently bonded to insoluble supports as described in U.S. Patent Nos. 3,551,555, 3,553,310, 4,048,298, and RE-29,474. Binding of antibodies to polystyrene by adsorption has been described in U.S. Patents Nos. 3,646,346 and 4,092,408, for example. Allergens have been covalently bonded to a variety of insoluble supports as described in U.S. Patents Nos. 3,720,760.

Polyethylene glycol has been used in protein fractionation processes as described by A. Polson et al, Biochim. Biophys. Acta., vol. 82, pp 463-475 (1964) and A. Polson et al, Vox Sang, vol. 23, pp 107-118 (1972).

This invention relates to a method for identifying and quantifying allergen specific $IgG_4$ levels in patient serum. It comprises the steps of

(a) providing an insoluble support having allergen adhering thereto, wherein the insoluble support has a plurality of reaction wells separated by opaque material;

(b) contacting the insoluble support with patient serum for a sufficient time to permit conjugation and removing the patient serum therefrom;

(c) contacting the insoluble support with a solution containing anti-$IgG_4$ antibody labelled with a fluorigenic enzyme for a time between 30 and 180 minutes and removing the solution containing any unconjugated anti-$IgG_4$ antibody therefrom;

(d) contacting the insoluble support with a solution of a substrate which undergoes reaction in the presence of the fluorigenic enzyme to yield fluorescent product for a time between 5 and 240 minutes;

(e) measuring the fluorescence level in the solution; and

(f) comparing the fluorescence level measured in step (e) with fluorescence levels of control solutions to determine the concentration of allergen specific $IgG_4$ in the patient serum.

In the insoluble allergen reagent of this invention the allergen is preferably adherent to the insoluble support by non-covalent bonding such as by absorption or adsorption, fur example. The allergen adhering to the insoluble support is preferably present as an allergen covalently bonded to a water-soluble polymer having an absorption or adsorption affinity for the insoluble support. The allergen-polymer product is adherent to the insoluble support by non-covalent bonding.

The insoluble support has a plurality of test wells separated by opaque material. Preferably, the anti-$IgG_4$ antibody is a monoclonal antibody to which alkaline phosphatase is bound, the anti-$IgG_4$ is contacted with the insoluble support in an aqueous solution containing from 1 to 8 weight percent polyethylene glycol having a molecular weight of from 1000 to 10,000 and a non-ionic surfactant, and the substrate is 4-methylumbelliferyl phosphate. If the allergen bonded to the insoluble support is covalently bonded to a water-soluble polymer having absorption or adsorption affinity for the insoluble support, in a preferred procedure the insoluble support is prerinsed with an aqueous buffered rinse solution containing from 0.0001 to 0.5 weight percent of the water-soluble polymer.

The role of $IgG_4$ in immune response is not yet clearly understood. Halpern, G.M. Clin. Rev. Allerg. 1:303-308 (1983) notes their experimental data shows that $IgG_4$ levels increase concurrently with desensitization of allergic specific hypersensitivity, supporting a hypothesis that desensitization is effected by increasing allergen specific $IgG_4$ levels.

Key to successful treatment of allergic conditions is the accurate identification of the offending allergen and the titration of the patient to determine the desensitization dosage. In general, reconstituted allergen extract is injected in sufficient quantity to cause major production of antigen-specific IgG (blocking antibody) and major production and/or activation of suppressor T lymphocytes. Monitoring $IgG_4$ levels is believed to be important in determining the dosage quantities.

The method of this invention provides the specificity and accuracy to monitor allergen specific $IgG_4$ levels in patient serum, providing valuable information relating to desensitization treatment regiments. With the method of this invention, more exact assessment of the suitable desensitization dose can be determined. Booster injections can be timed to maintain the requisite $IgG_4$ levels. Usually the doses required for booster injections are substantially greater than the maximum dose required for control of the initial allergic reaction.

The process of this invention comprises a first step of contacting an insoluble support having allergen adhering thereto with patient serum for a sufficient time to permit conjugation of allergen with $IgG_4$ in the patient serum and then removing the patient serum from the support. In this procedure the patient serum is preferably undiluted prior to contact with the supported allergen. The incubation time should be sufficient to permit substantial conjugation to occur, the time being temperature dependent. Suitable incubation times are from 30 to 180 minutes at temperatures within the range of from 18 to 40°C, the preferred contact time being from 60 to 120 minutes at temperatures within the range of from 20 to 26°C.

The insoluble support, which consists of a plurality of wells separated by opaque material, having the allergen adhering thereto is an important aspect of this invention. The allergen can be any allergenic material such as allergen derived from pollens derived from trees, shrubs, weeds, and grasses; molds; smuts; dusts; allergens derived from danders, hair, and epidermals of animals; extracts derived from insects including insect venoms; and from foods.

A wide variety of compounds can be employed as the solid support, the primary consideration being the binding of the allergens to the surface, the absence of interference with the enzyme labeled anti-IgG$_4$ antibody reagent, enzymatic reaction thereof with a substrate and fluorescent properties of the enzymatic reaction product. Organic and inorganic polymers, both natural and synthetic can be employed as the solid support. Examples of suitable polymers include polyethylene, polypropylene, polybutylene, poly(4-methylbutylene), butyl rubber and other synthetic rubbers, silicone rubbers and silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, styreneacrylonitrile copolymers, rayon, nylon, polyvinylbutyrate, polyformaldehyde, etc. Other materials which can be employed as the insoluble support are silica gel, silicon wafers, glass, paper, insoluble protein, metals, metaleoids, metal oxides, magnetic materials, semi-conductive materials, cermets or the like. In addition are included substances that form gels, such as proteins such as gelatins, lipopolysaccharides, silicates, agarose, polyacrylamides or polymers which form several aqueous phases such as dextrans, polyalkylene glycols (alkylene with 2 to 3 carbon atoms) or surfactants, e.g. amphophilic compounds such as phospholipids, long chain (12-24 carbon atoms) alkyl ammonium salts and the like.

A preferred diagnostic support of this invention comprises a polystyrene, styrene copolymers including styrene-(vinyl monomer) copolymers such as styreneacrylo nitrile copolymers, or polyolefins such as polyethylene and polypropylene, and acrylate and methacrylate polymers and copolymers. The allergenic extract is preferably bound thereto by adsorption, ionic bonding, van der Waals adsorption, electrostatic bonding, other non-covalent bonding. It can also be bound to the support by covalent bonding. A particularly advantageous support for this procedure comprises a microtiter plate having a plurality of wells. The well surface or plastic cup inserts therein can constitute the allergen support. The microtiter plate or the well inserts are opaque to light so that excitation light applied to a well or fluorescence generated in response thereto does not reach or influence contents of the surrounding wells. With this system each well can be employed as a test system independent of the other wells.

Preferable the allergen is covalently bonded to a water-soluble polymer having an affinity for the insoluble substrate. The allergen-polymer product is then adhered to the insoluble substrate by non-covalent bonding such as adsorption or absorption.

Suitable water-soluble proteins include bovine serum albumins of bovine (BSA), human (HSA), rabbit (RSA), goat (GSA), sheep (SSA), horse (HOSA), etc.; serum gamma Globulin of the previously described animals; and other animal proteins such as ovalbumin, fribrinogen, thrombin, transferin, glycoproteins, etc. Suitable water-soluble amino acid polymers include polylysine, polyglutamic acid, polyalanine, polyhistidine, polymethionine, polyproline, etc. The allergen can be covalently bonded to water-soluble protein or amino acid polymer with conventional coupling agents using methods which are known in the art. Preferably the coupling agent is a carbodiimide such as 1-ethyl-3-(3-N,N-dimethylaminopropyl)carbodiimide hydrochloride and 1-cyclohexyl-3-(2-morpholinoethyl)carbodiimide methyl-p-toluenesulfonate. Other suitable coupling agents include aldehyde coupling agents having either ethylenic unsaturation such as acrolein, methacrolin, or 2-butenal or having a plurality of aldehyde groups such as glutaraldehyde, propanedial or butanedial. Other coupling agents include bifunctional NHS-esters such as disuccinimidyl suberate, disuccinimidyl tartarate, bis[2-(succinimidooxycarbonyloxy)ethyl)sulfone, disuccinimidyl(N,N'-diacetylhomocystine, d ithio-bis(succinimidylpropionate), ethylene glycolbis(succinimidyl succinate); hetero-bifunctional reagents such as N-5-azido-2-nitrobenzoyloxy succinimide, p-azidophenacyl bromide, p-azidophenylglyoxal, 4-fluoro-3-nitrophenyl azide, N-hydroxysuccinimidyl-4-azidobenzoate, m-maleimidobenzoyl N-hydroxysuccinimide ester, methyl-4-azidobenzoimidate HCl, p-nitrophenyl 2-diazo-3,3,3-trifluoropropionate, N-succinimidyl-6(4'-azido-2'-nitrophenylamino)hexanoate, succinimidyl 4-(N-maleimido-methyl)cyclohexane-1-carboxylate, succinimidyl 4-(p-maleimidophenyl)-butyrate, N-succinimidyl (4-azidophenyldithio)propionate, N-succinimidyl 3-(2-pyridyldithio)-propionate, N-(4-azidophenylthio)phthalimide, homobi-functional reagents such as 1,5-difluoro-2,4-dinitrobenzene, 4,4'-difluoro-3,3'-dinitrodiphenylsulfone, 4,4'-diisothio-cyano-2,2'-disulfonic acid stilbene, p-phenylenediisothio-cyanate, carbonylbis(L-methionine p-nitrophenyl ester), 4,4'-dithiobisphenylazide, erythritolbiscarbonate; and bifunctional imidoesters such as dimethyl adipimidate 2HCl, dimethyl suberimidate, dimethyl 3,3'-dithiobispropion-imidate 2HCl, 2-iminothiolane HCl, covalent bonding of allergen to the insoluble protein can be carried out with the above reagents by conventional, well-known reactions, for example in the aqueous solutions at a neutral ph, at temperatures of less than 10°C for 18 hours or overnight.

In a procedure for non-covalent adhesion of allergen to the surface of an insoluble support, the allergenic material can be applied to the surface of a support such as a polystyrene microtiter well or polystyrene individual insert well therefor, in an aqueous buffer solution. The surface is initially cleaned with a cleaning fluid such as methanol and dried. The buffered allergen solution is placed in the well or insert cup and incubated at room temperature until adsorption occurs, for example for from 2 to 18 hours and preferable from 16-18 hours, at temperatures of from 4 to 40°C and preferable from 20 to 26°C. The well is then rinsed with a weak saline solution and dried. Other procedures for covalently adhering allergens to insoluble supports are described by Ichiro Chibata in Immobilized Enzymes, Halsted Press, New York, 1978, and by A. Cuatrecasas, J. Bio. Chem. 245 3059(1970), the entire contents of which are hereby incorporated by reference. The surface can be coated with a protein and coupled with allergen using the procedures described in U.S. patent 4,210,418 using glutaraldehyde as a coupling agent, for example. In a still further procedure, the well can be coated with a layer having free isocyanate groups such as a polyether isocyanate, and application of the allergen in aqueous solution thereto effects the requisite bonding. In a still further procedure, the allergen can be coupled to a hydroxylated material by means of cyanogen bromide as described in U.S. Patent No. 3,720,760.

Preferred allergens are those described in European Patent Application 83110233.0, (EP-A-107832).

If the allergen is covalently bonded to a water-soluble polymer having an affinity for the insoluble substrate and the water-soluble polymer has antigenic properties, the first step is preferably preceded by a prerinse step. In the prerinse step, the support surface is contacted with an aqueous buffered rinse solution containing from 0.001 to 0.5 weight percent of the water-soluble antigenic polymer to which the allergen is bound. This prerinse step is particularly advantageous when the water-soluble polymer is water-soluble animal protein because rinse residue will provide a sufficient amount of water-soluble protein to conjugate with any of the protein-specific $IgG_4$ which is present in the patient serum. The protein-specific $IgG_4$ would otherwise complex with the protein in the insoluble support as a non-specific $IgG_4$ binding, greatly reducing the sensitivity of the assay.

A preferred rinse solution of this invention is an aqueous phosphate buffer solution having a phosphate molarity of from 0.01 to 0.05, a pH of from 6 to 8 and containing from 0.01 to 0.01 weight percent non-ionic surfactant and from 0.0001 to 0.5 weight percent of the antigenic protein to which the allergen is coupled. Suitable non-ionic surfactants include polyoxyethylene ethers (BRIJ) such as lauryl, cetyl, oleyl, stearyl, and tridecyl polyoxyethylene ethers; polyoxyethylenesorbitans (TWEEN) such as polyoxyethylenesorbitan monolaurate, monopalmitate, monostearate, monoleate and trioleates; and other polyoxyethylene ethers (TRITON), for example. A preferred non-ionic surfactant is octylphenoxypolyethoxy ethanol having 40 ethylene oxide units (TRITON® X-405, Rohm and Haas Company).

The buffer solution is advantageously prepared from a reagent concentrate of the invention comprising from 0.005 to 2.5 weight percent of the animal protein corresponding to the animal protein to which the allergen is covalently bonded, from 0.5 to 5 weight percent non-ionic surfactant, from 10 to 20 weight percent sodium chloride, from 0.5 to 5 weight percent stabilizer and sufficient phosphate salt to provide for a 0.02 to 0.05 M phosphate solution. The pH can be from 6 to 8. The preferred buffer concentrate contains about 0.5 weight percent of the animal protein, 0.1 weight percent TRITON® X-405 non-ionic surfactant, 17 weight percent sodium chloride, and 2 weight percent sodium azide, 0.01 M phosphate and has a pH of 7.4.

After conjugation of serum $IgG_4$ with allergen adhering to the insoluble support has occurred, the patient serum is removed therefrom. Surplus liquid is removed and the solid surface is then rinsed with a suitable rinse solution such as that described above.

The second step of the process of this invention comprises contacting the insoluble support with an anti-$IgG_4$ antibody labeled with a fluorogenic enzyme. The incubation is continued for sufficient time to permit serum $IgG_4$ conjugated with allergen (if any) an the insoluble support to conjugate with the anti-$IgG_4$ antibody. After incubation, the excess liquid is removed, and the surface of the insoluble support is rinsed with a weak saline solution as described above with respect to the first step to remove unconjugated antibody. Preferably the support is rinsed with the preferred rinse solution of this invention described above.

Anti-$IgG_4$ antibodies are available from many sources, and the methodology for producing them is well known and is described in several of the patents and publications cited above. The preferred antibodies are monoclonal antibodies. The technology for making monoclonal antibodies is well developed, and the procedures suitable for making monoclonal anti-$IgG_4$ antibodies are described by D. Catty, et al in "Antisera in Immunoassays with special Reference to Monoclonal Antibodies to Human Immunoglobulins", Immunoassay's for the 80's, supra, pp 133-153 and the publications cited therein, the entire contents of which are hereby incorporated by reference.

Fluorogenic enzymes and methods for bonding them to antibodies without impairing the ability of the antibody to selectively conjugate with antigen are well known in the art. Suitable enzymes and procedures for coupling them to antibodies are described in U.S. patent no. 4,190,496, for example the contents thereof being hereby incorporated by reference. The preferred fluorogenic enzymes and the suitable substrates corresponding thereto include horse-radish peroxidase for which a suitable substrate is homovanillic acid or 4-hydroxy-3-methoxyphenylacetic acid, beta-galactosidase for which a suitable substrate is 4-methylumbelliferyl-beta-D-galactoside, alkaline phosphatase for which a suitable substrate is 4-methylumbelliferyl phosphate and other umbelliferyl phosphates such as 4-carboxyumbellifery phosphate, and umbelliteryl phosphate 4-carboxy alkylesters, etc.

Examples of suitable procedures for enzyme labeling the anti-IgG$_4$ antibody include carbodiimides, dialdehyde, and bifunctional coupling reagents as described in covalent linkage of allergen to water-soluble polymer or periodate coupling where enzyme has glycoprotein moiety.

The enzyme labeled anti-IgG$_4$ antibody is applied to tile insoluble support in an aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as a polyoxyethylene sorbitan ester employed in the rinse solutions described above. The rinse solutions described herein can also be used.

A preferred solution of this invention comprises from 0.1 micrograms per ml to 5 micrograms per ml and preferably from 1 microgram per ml to 2 microgram per ml anti-IgG$_4$ antibody in an aqueous phosphate buffered solution having a phosphate molarity of from 0.005 to 0.1 and preferably from 0.01 to 0.05 and a pH of from 6.0 to 8.0 and preferably 7.2 to 7.6. A critical ingredient in the anti-IgG$_4$ solution is polyethylene glycol having molecular weights of from 1000 to 8000 and preferably from 2000 to 4000 in concentrations of from 1 to 8 and preferably from 2 to 6 weight percent. Polyethylene glycols greatly increase the speed and sensitivity of the reaction. Another important ingredient is a non-ionic surfactant in concentrations of from 0.01 to 0.5 and preferably from 0.02 to 0.1 weight percent. Suitable non-ionic surfactants include those described above with respect to rinse solutions, for example. A preferred non-ionic surfactant is TRITON® X-405. The surfactant surprisingly reduces the non-specific background fluorescence signal in the assay.

With the preferred anti-IgG$_4$ solutions of this invention, the incubation time of the solutions with the insoluble support is temperature dependent. At temperatures of 18 to 40°C, incubation times of at least from 30 to 180 minutes can be used. The preferred temperatures are within the range of from 20 to 26°C, and at these temperatures, incubation times from 60 to 120 minutes can be employed. It should be appreciated that prolonged incubation times in any of the steps of this invention can reduce the efficiency of the process. Since rapid analysis is an objective of this invention, the lowest times which still yield the desired accuracy are preferred.

The solid support is then rinsed to remove residual, unconjugated enzyme labeled anti-IgG$_4$ antibody. The rinse solutions described above are suitable.

The third step of the process of this invention comprises contacting the solid support with a solution of a substrate which undergoes chemical reaction in the presence of the fluorogenic enzyme for a time sufficient for fluorescent compounds to be formed. Suitable substrates and the enzymes they are converted by are known in the art and are described in U.S. patent no. 4,190,496, for example. Examples of substrates have been described hereinabove with respect to the corresponding fluorogenic enzyme.

The solid is contacted with an aqueous solution of the substrate containing from $10^{-2}$ to $10^{-10}$ molar and preferably from $10^{-4}$ to $10^{-5}$ molar concentrations of the substrate. Preferred additional reagents and buffers in the substrate solution include 2-amino-2-methyl-1-propanol buffer and magnesium chloride, for example.

The substrate solution is incubated with the insoluble support for sufficient time for the fluorescent reaction product to form. At temperatures of from 18 to 40°C, incubation times of from 5 to 240 minutes can be used. Preferably, the temperature is within the range of from 20 to 26°C, and the incubation time is from 30 to 90 minutes.

The equipment and procedures for determining the level of fluorescence in the substrate solutions are those conventionally employed in the art. The level of fluorescene is a function of the enzyme concentration on the insoluble support which is, in turn, a function of the allergen specific IgG$_4$ level in the patient serum. By comparing the fluorescence level with the levels measured by carrying out the procedure with control solutions containing known concentrations of the respective allergen specific IgG$_4$, the precise concentration of the corresponding IgG$_4$ antibody in the patient serum can be determined.

Suitable fluorometers are the fluorometers by Perkin-Elmer, American Instrument Company, and Turner Designs. The Allergenetics Fluorometer (Allergenetics, Inc., Mountain View, California) is preferred.

This invention is further illustrated by the following specific but non-limiting examples. Temperatures are given in degrees Centigrade and concentrations are given as weight percents unless otherwise specified.

EXAMPLE 1

To a solution of timothy grass pollen allergen extract (3 mg/ml) was added 10 microliters of a 5 wt. %

bovine serum albumin (BSA) solution. After addition, the solution was kept at 4°C, and 5 mg of 1-Ethyl-3-(3-N, N-Dimethyl-aminopropyl) carbodiimide (ECDI) was added. The mixture was gently stirred at 4°C for 20 minutes. The additions of both BSA and ECDI were repeated three more times. The final mixture was allowed to stand at 4°C overnight to yield a conjugate of timothy grass pollen allergen covalently bonded to BSA.

The timothy grass pollen allergens-conjugate with BSA prepared in Example 1 was diluted in phosphate buffer, pH 8.5. To each microtiter well was added precisely 100 microliters of the diluted conjugate solution. The coating process was allowed to proceed at room temperature for 2 hours (or overnight). At the end of the coating process, the liquid in each well was removed by aspiration. The wells were washed three times (3x200 microliters) with a phosphate washing buffer obtaining sorbitol and TRITON® X-405. The well thus coated can be used for assaying patient serum for timothy grass pollen allergen specific IgG$_4$ antibodies.

EXAMPLE 2

The procedure of Example 1 was repeated, replacing the timothy grass pollen extract with the following allergenic extracts to yield microwells having the corresponding allergens adhering to the surface thereof.

Grasses - Bermuda Grass, Cynodon dactylon, Orchard Grass, Dactylis glomerata, Perennial Rye Grass, Lolium perenne, June Grass (Kentucky Blue), Poa pratensis, Bent Grass, Agrostis maritima, Johnson Grass, Sorghum halepense, Brome Grass, Bromus inermis, Bahia Grass, Paspalum notatum, Corn Grass, Zea mays, Meadow Fescue, Festuca elatior, and Redtop, Agrostis alba;

Weeds - Short Ragweed, Ambrosia artemisifolia, Western Ragweed, Ambrosia psilostachya, False Ragweed Franseria acanthicarpa, Sagebrush (common), Artemisia tridentata, Dandelion, Taraxacum vulgare, English Plantain, Plantago lanceolata, Lamb's Quarters, Chenopodium album, Russian Thistle, Salsola kali, Goldenrod, Solidago sp., Pigweed, Amaranthus retroflexus, Dock (yellow), Rumex crispus, and Sheep Sorrel, Rumex acetosella;

Trees - Box Eider (Maple), Acer negundo, Alder, Alnus rhombifolia, Birch, Betula nigra, Mountain Cedar, Juniperus sabinoides, White Oak, Quercus alba, Elm, Ulmus americana, Olive, Olea europaea, Black Walnut, Juglans nigra, Sycamore, Platanus occidentalis, Cottonwood, Populus trichocarpa, White Ash, Fraxinus americana, White Pine, Pinus monticola, Eucalyptus, Eucalyptus sp., Acacia, Acacia baileyana, Aspen, Populus tremuloides, Arizona Cypress, Cupressus arizonica, Mesquite, Prosopis juliflora, Privet, Ligustrum ovalifolium, Melaleuca (Punk Tree), Melaleuca leucadendron, and Australian Pine (Beefwood), Casuarina equisetifolia;

Epidermals - Cat Epithelium, Dog Hair and Dander, Horse Hair and Dander, Cow Hair and Dander, Guinea Pig Hair and Dander, Feather Mix (Chicken, Duck, & Goose), and Wool (Sheep);

Molds - Penicillium notatum, Cladosporium herbarum, Aspergillus fumigatus, Mucor racemosus, Candida albicans, and Alternaria tenuis;

House Dust; Mite - Dermatophagoides farinae; and

Foods - Milk, Wheat, Corn, Rice, Peanut, Soybean, Shrimp, Tomato, Pork, Carrot, Orange, Potato, Tuna, Beef, Lamb, Chicken, Whole Egg, Yeast (Bakers), Sweet Potato, Cabbage, Lettuce, Pepper (Bell), Apple, Cranberry, Grape, Barley, and Onion. This yielded the corresponding, respective, covalently bonded BSA conjugate of each allergen;

Tree Pollens - Acacia-Acacia longifolia; Ailanthus (See Tree of Heaven) - Ailanthus altissima; Alder, Mountain (Tag) (Slender) - ainus tenuifolia/incana; Alder, Red (Oregon) - Alnus rubra; Alder, Sitka - Alnus sinuata; Almond - Prunus amygdalus; Apple - Pyrus malus (Malus pumila); Apricot - Prunus armeniaca; Arbor Vitae, Oriental (Ornamental) - Betula papyrifera; Birch, Spring - Betula fontinalis; Birch, White (Weeping) - Betula pendula; Birch, Yellow - Betula lutea; Blue Beech (Am. Hornbeam) - Carpinus carolineana; Bottle Brush - Callistemon citrinus; Butternut - Juglans cinerea; Carob Tree - Ceratonia siliqua; Cedar, Deodar - Cedrus deodora; Cedar, Giant - Thuja plicata; Cedar, Incense -Linocedrus decurrnes; Cedar, Japanese - Cryptomeria japonica; Cedar, Port Orford (Lawson Cypress) - Chamaecyparis lawsoniana; Cedar, Red - Juniperus virginiana; Cedar, Rocky Mountain - Juniperus scopulorum; Cedar, Salt (Tamarisk) - Tamarix gallica; Cedar, White - Thuja occidentalis; Cherry, Prunus cerasus; Chestnut, American - Castanea dentata; Chestnut, Horse - Aesculus hippocastanum; Cottonwood, Common - Populus deltoides; Cottonwood, Fremont - Populus fremontii; Cypress, Bald (White) - Taxodium distichum; Cypress, Italian - Cupressus sempervirens; Cypress, Monterey - Cupressus macrocarpa; Elderberry - Sambucus glauca; Elm, Cedar (Fall Blooming) - Ulmus crassifolia; Elm, Chinese - Ulmus parvifolia; Elm, Siberian - Ulmus pumila; Elm, Slippery - Ulmus fulva (rubra); Fir, Douglas - Pseudotsuga menziesii; Fir, Red (Noble) - Abies nobllis (procera); Fir, White - Abies concolor; Gum, Sweet-Liquidambar styraciflua; Hackberry - Celtis occidentails; Hazelnut, American - Corylus americana; Hemlock, Eastern-Tsuga canadensis; Hemlock, Western - Tsuga heterophylla; Hickory, Shagbark - Carya ovata; Hickory, Shellbark - Carya laciniosa; Hickory, White - Carya tomentosa; Ironwood (Hop-Hornbeam) - Ostrya virginiana; Juniper, California - Juniperus californica; Juniper, Chinese - Juniperus chinensis; Juniper, Oneseed - Juniperus monosperma; Juniper, Pinchot - Juniperus pinchotti; Juniper, Utah - Juniperus osteosperma (juniperus utahensis); Juniper, Western - Juniperus occidentalis; Lilac - Syringa vulgaris; Linden (Basswood) - Tilia americana; Locust, Black - Robinia pseudoacacia; Maple, Big-Leaf (Coast) - Acer macrophyllum; Maple, Hard (Sugar) - Acer saccharum; Maple, Red - Acer rubrum; Maple, Soft (Silver) - Acer saccharinum; Mock Orange, Wild (Syringa) - Philadelphus lewisii; Mulberry, Paper - Broussonetia papyifera; Mulberry, Red - Morus rubra; Mulberry, White - Morus alba; Oak, Arizona (Gambel) - Quercus gambelii; Oak, Arizona Scrub (Canyon) - Quercus chrysolepsis; Oak, Black (Yelow) - Quercus velutina; Oak, Black Jack - Quercus marilandica; Oak, Bur - Quercus macrocarpa; Oak, California Black - Quercus kelloggii-californica; Oak, California Scrub - Quercus dumosa; Oak, Coast Live - Quercus agrifolia; Oak, Engelmann - Quercus engelmanii; Oak, Garry (Western White) - Quercus garryana; Oak, Holly - Quercus ilex; Oak, Interior Live - Quercus wislizenii; Oak, Post - Quercus stellata; Oak, Red - Quercus rubra; Oak, Swamp (Pin) - Quercus palustris; Oak, Valley - Quercus lobata; Oak, Virginia Live - Quercus virginiana; Oak, Water - Quercus nigra; Olive - Olea europaea; Orange - Citrus sinensis; Osage Orange - Maclura pomifera; Palm, Date - Phoenix dactylifera; Palm, Dwarf - Chamaerops humulis; Palm, Canary Island Date (Ornamental) - Phoenix canariensis; Palm, Queen - Cocos plumosa; Peach - Prunus persica; Pear - Pyrus communis; Pecan - Carya pecan; Pepper Tree, California - Schinus molle; Pepper Tree, Brazilian - Schinus terebinthifolius; Pine, Austrian - Pinus nigra; Pine, Canary Island - Pinus canariensis; Pine, Digger - Pinus sabiniana; Pine, Loblolly - Pinus taeda; Pine, Lodgepole - Pinus contorta; Pine, Monterey - Pinus radiata; Pine, Pinyon - Pinus edulis; Pine, Red (Norway) - Pinus resinosa; Pine, Shortleaf - Pinus echinata; Pine, Virginia Scrub - Pinus virginiana; Pine, Western Yellow (Ponderosa) - Pinus ponderosa; Pine, White (Eastern) - Pinus strobus; Plum (Prune) - Prunus domestica; Poplar, Balsam - Populus balsamifera; Poplar, Lombardy - Populus nigra-italica; Western Balsam (See Cottonwood, Black) Populus trichocarpa; Poplar, White - Populus alba; Privet - Ligustrum spp.; Redwood - Sequoia sempervirens; Russian Olive - Elaeagnus angustifolia; Spruce, Red - Picea rubens; Spruce, Sitka - Picea sitchensis; Sycamore, Mapleleaf - Platanus acerifolia; Sycamore, Western - Platanus racemosa; Tamarack (Larch) - Larix occidentalis; Tamarisk (see Cedar, Salt) - Tamarix gallica; Tree of Heaven - Ailanthus altissima; Walnut, Arizona - Juglans rupestris; Walnut, Hind's California Black - Juglans hindsii; Walnut, So. California Black - Juglans californica; Walnut, English - Juglans regia; Willow, Arroyo - Salix lasiolepis; Willow, Black - Salix nigra; Willow, Pussy - Salix discolor; Willow, Red - Salix laevigata; Willow, Yellow - Salix lasiandra.

Grass and Weed Pollens - Barley, Cultivated - Hordeum vulgare; Bluegrass, Annual - Poa annua; Bluegrass, Canada - Poa compressa; Bluegrass, Sandberg - Poa sandbergii; Brome Broncho-Ripgut - Bromus rigidus; Brome, California -Bromus carinatus; Brome, Cheat - Bromus secalinus; Brome, Soft Cheat - Bromus mollis; Bunch, Blue (Northwestern Bunch) - Agropyron spicatum; Canarygrass - Phalaris canariensis; Canarygrass, Reed -Phalaris arundinacea; Fescue, Red -Festuca rubra; Grama Grass, Blue (Side Oats) - Bouteloua gracilis; Koeler's Grass (Western Junegrass) -Koeleria cristata; Lovegrass, Hawaiian - Eragrostis variabilis; Oats, Common Cultivated -Avena sativa; Oatgrass, Tall - Avena elatior (Arrhenatherum elatius); Quack Grass - Agropyron repens; Rye, Cultivated -Secale cereale; Ryegrass, Alkali - Elymus triticoides; Ryegrass, Giant Wild - Elymus cinereus; Ryegrass, Italian - Lolium multiflorum; Ryegrass, Western - Elymus glaucus; Salt Grass - Distichlis stricta; Sorghum, Common Cultivated - Sorghum vulgare; Sudan Grass -Sorghum vulgare var. sudanese; Sweet Vernal grass - Anthoxanthum odoratum; Velvetgrass - Holcus lanatus; Wheat, Cultivated - Triticum aestivum; Wheatgrass, Crested - Agropyron cristatum; Wheatgrass, Western - Agropyron smithii; Alfalfa - Medicago sativa; Aster - Aster sinensis; Balsam Root - Balsamorhiza sagittata; Bassia - Bassia hyssopifolia; Beach Bur - Franseria bipinnatifida; Burro Brush (Greasebush) - Hymenoclea salsola; Careless Weed - Amaranthus palmeri; Castor Bean - Ricinus communis; Cattail, Broadleaf - Typha latifolia; Clover, Red - Trifolium pratense; Clover, Sweet, YellowMelilotus officinalis; Clover, White (Dutch) - Trifolium repens (album); Cocklebur, Common - Xanthium strumarium; Cocklebur, Spiny - Xanthium spinosum; Cosmos - Cosmos bipinnatus; Daffodil - Narcissus pseudo-narcissus; Dahlia - Dahlia pinnata x coccinea; Daisy/ Chrysanthemum (Oxeyed Daisy) - Chrysanthemum leucanthemum; Dock, Bitter - Rumex obtusifolius; Dog Fennel (Mayweed) - Anthemix cotula; Fireweed, Alaska - Epilobium angustifolium; Gladiolus - Gladiolus Xhortulanus; Greasewood - Sarcobatus vermiculatus; Hemp - Cannabis sativa; Hops - Humulus lupulus; Hopsage - Grayia spinosa; Iodine Bush (Burro Weed) - Allenrolfea occidentalis; Kochia (Mex. Firebush) - Kochia scoparia; Lily, Easter - Lilium longiflorum; Marigold - Tagetes patula; Marshelder, Burweed (Giant Poverty) - Iva Xanthifolia; Marshelder, Narrowleaf (August) - Iva angustifolia; Marshelder, True (Rough) - Iva ciliata; Mexican Tea -Chenopodium ambrosioides; Mustard, Black - Brassica nigra; Mustard, Common Yellow - Brassica campestris; Nettle - Urtica dioica (gracilis); Pickleweed - Salicornia ambigua; Pigweed, Spiny - Amaranthus spinosus; Poppy, California - Eschoscholzia Californiea Povertyweed, Small - Iva axillaris; Rabbit Brush - Chryso-thamnus nauseosus; Rabbit Bush (Bur Ragweed) - Franseria deltoides; Ragweed, Canyon - Franseria ambrosioides; Ragweed, Desert - Franseria dumosa; Ragweed, Giant - Ambrosia trifida; Ragweed, Silver - Dicoria canescens; Ragweed, Slender - Franseria tenuifolia; Ragweed, Southern - Ambrosia bidentata; Rose - Rosa multiflora; Sagebrush - Annual - Artemisia annua; Sagebrush, Coast - Artemisia californica; Sagebrush, Green (Tarragon)-Artemisia dracunculus; Sagebrush, Mugwort - Artemisia vulgaris heterophylla; Sagebrush, Pasture (Carpet) - Artemisi frigida; Sagebrush, Sand Dune - Artemisia pycnocephala; Sagebrush, White (Prairie) - Artemisia ludoviciana; Saltbush, Annual - Atriplex wrightii; Scale, All - Atriplex polycarpa; Scale, Bract - Atriplex serenana bracteosa; Scale, Brewers - Atriplex lentiformis breweri; Scale, Lens -Atriplex lentiformis; Scale, Red - Atriplex rosea; Scale, Silver (Fogweed) - Atriplex argentea expansa; Scale, Spear - Atriplex patula hastata; Scale, Wing (Shad) -Atriplex canescen; Scotch Broom - Cytisus scoparius; Sea Blite, California - Suaeda californica; Sedge - Carex barbara; Sheep Fat - Atriplex confertifolia; Snapdragon - Antirrhinum majus; Suaeda (See Sea Blite); Sugar Beet - Beta vulgaris; Sunflower - Helianthus annuus; Waterhemp, Western - Acnida tamariscina; Winter Fat - Eurotia lanata; Wormseed (Jerusalem Oak) - Chenopodium botrys; Wormwood, Absinthe - Artemisia absinthium;

Epidermals and Glandular Extracts - Camel Hair and Dander; Deer Hair and Dander; Feathers, Parakeet; Feathers, Pigeon; Feathers, Turkey; Fox Fur; Gerbil Hair & Epithelium; Glue, Fish; Goat Hair & Dander; Hamster Hair & Epithelium; Hog Hair & Dander; Human Hair; Mink Fur; Mohair; Monkey Hair & Epithelium; Mouse Hair & Epithelium; Poodle Hair & Dander; Pyrethrum; Rabbit Hair & Epithelium; Rat Hair & Epithelium; Seal Fur; Wool, Sheep;

Dusts - Acacia Gum; Alfalfa Hay; Algae, Chlorella spp.; Carragheen Gum; Coconut Fiber; Cotton Linters; Cottonseed; Dust, Barley; Dust, Corn; Dust, Grain Mill; Dust Mattress; Dust, Oat; Dust, Pea; Dust, Rye; Dust, Soybean; Dust Upholstery; Dust, Wheat; Dust, Wood - Cedar/Juniper; Dust, Wood - fir/Hemlock; Dust, Wood - Gum; Dust, Wood - Mahogany; Dust, Wood - Maple; Dust, Wood - Oak Mix; Dust, Wood - Pine Mix; Dust, Wood - Redwood; Dust, Wood - Spruce; Dust, Wood - Walnut; Fern Spores, sp.; Flax Fiber; Flaxseed; Hemp; Jute; Kapok; karaya Gum; Lycopodium; Orris Root; Paper Mix; Pyrethrum; Silk; Sisal; Tragacanth Gum; Timothy Hay; Tobacco, Pipe; Tobacco, Cigarette; Tobacco, Cigar; Tobacco, Leaf;

Foods - Allspice; Almond; Apricot Food; Arrowroot; Artichoke; Asparagus; Avacado; Banana; Bay Leaf; Bean, Kidney; Bean, Lima; Bean, Navy; Bean, Pinto-Frijole; Bean, String/Wax; Beet; Black-Eyed Pea; Blueberry; Brazil Nut; Buckwheat; Cashew Nut; Celery; Cheese, Cheddar (American); Cheese, Parmesan; Cheese, Roquefort; Cheese, Swiss; Cherry Mix; Chewing Gum Base; chicken, Chicory; Chili Pepper; Chocolate/Cocoa; Cinnamon; Clam; Cloves; Cola; Coconut; Codfish Mix; Coffee; Crab; Cucumber; Curry Powder; Date; Dill; Egg White; Egg, Yolk; Eggplant; Endive; Garlic; Gelatine; Ginger; Raisin Mix; Grapefruit; Haddock; Halibut; Hazelnut; (Filbert); Herring; Honey; Hops Food; Horseradish; Lamb; Lemon; Lentil; Lime; Liver, Beef (Calves); Lobster; Mackerel; Malt; Mangoes; Maple, Syrup/Sugar; Melon, (see Muskmelon Mix); Milk, Cow's (Albumin); Milk, Cow's (Casein); Milk, Cow's (Whey); Milk, (Evaporated); Milk, Goat's; Mint Mix (Peppermint/Spearmint); Mushroom; Mustard; Nutmeg; Oat, Whole (Grain); Okra; Olive Mix; Onion; Orange, Mandarin/Tangerine; Oregano; Oyster Mix; Papaya; Paprika; Parsley; Parsnip; Pea; Peach Food; Pear Food; Pecan Food; Pepper, Black/-White; Pepper, Bell (Green/Red); Perch, Lake; Pineapple; Plum/Prune Mix; Poppy Seed; Pumpkin; Rabbit Meat; Radish; Raspberry; Snapper, Rhubarb; Rice, Wild; Rye, Whole (Grain); Safflower Seed; Sage; Salmon; Scallops; Sesame Seed; Sole; Spinach; Squash, Mix; Strawberry; Sugar (Beet); Sugar (Cane); Sunflower Seeds; Tapioca; Tea; Thyme; Trout; Turkey; Turnip; Vanilla; Walnut Food, Black; walnut Food, English; Watermelon; Whitefish; Yeast, Brewers; Yeast Mix (Bakers/Brewers, Sacchoromyces cerevisiae);

Molds - Aspergillus clavatus; Aspergillus glaucus; Aspergillus nidulans; Aspergillus niger; Aspergillus restrictus; Aspergillus sydowi; Aspergillus terreus; Botrytis cinerea; Cephalosporium acremonium; Cephalothecium (Trichothecium) reseum; Chaetomium globosum; Cryptococcus terreus; Cunninghamella elegans; Curvularia spicifera; Dematium nigrum; Epicoccum nigrum; Epidermophyton floccosum; Fomes rimosus; Fusarium vasinfectum; Geotrichum candidum; Helminthosporium maydis; Helminthosporium; Hormodendrum (Cladosporium); Monilia sitophila; Mycogone sp.; Neurospora crassa; Nigrospora sphaerica; Oidiodendrum sp.; Paecilomyces varioti; Penicillium artramentosum; Penicillium biforme; Penicillium carminoviolaceum; Penicillium chrysogenum; Penicillium digitatum; Penicillium expansum; Penicillium glaucum; Penicillium intricatum; Penicillium luteum; Penicillium roqueforti; Penicillium roseum; Phoma herbarum; Pleospora sp.; Poria sp.; Pullularia pullulans; Rhizopus nigricans; Rhodotorula glutinis; Saccharomyces cerevisiae (See Yeast Mix); Scopulariopsis brevicaulis; Spondylocladium sp.; Sporobolomyces salmonicolor; Stemphylium botryosum; Streptomyces griseus; Trichoderma viride; Typhula idahoensis; Verticillum alboatrum;

Smuts - Smut, Barley; Smut, Bermuda; Smut Corn; Smut, Johnson; Smut, Oat; Smut, Sorghum; Smut, Wheat; and

Insects and Insect Venoms - Ants, (Black and Red); Ants, Carpenter; Ants, Fire; Aphid; Bee, Bumble; Bee, Honey; Blackfly; Butterfly; Caddis Fly; Cricket; Cockroach Mix; Deer Fly; Flea antigen; Fruit Flies; Gnat sp.; Hornet, Black and Yellow; Horse Fly; House Fly; Mayfly sp.; Mite (D. farinae); Mosquito Mix; Moth, Miller; Wasp; Yellow Jacket; Honey Bee Venom Protein - Apis mellifera; Wasp Venom Protein - Polistes sp.; White-faced Hornet Venom Protein - Dolichovespula maculata; Yellow Hornet Venom Protein - Dolichovespula arenaria; Yellow Jacket Venom Protein - Vespula sp.; Mixed Vespid Venom Protein.

EXAMPLE 3

A microtiter plate well product of Example 1 to which a BSA conjugate of timothy grass pollen allergen is adhered is contacted with patient serum containing timothy grass pollen allergen specific IgG$_4$ and incubated for 2 hours. The serum is removed, and the well washed three times with a buffered rinse solution containing 0.85 wt. % sodium chloride, 0.05 wt. % TRITON® X-405 and 0.1 wt. % sodium azide preservative in a 0.01 aqueous phosphate buffer solution, pH 7.2. Serum IgG$_4$ specific antibody for timothy grass pollen allergen is conjugated to the microtiter plate well surface.

The microtiter plate well is then contacted with 100 microliters of a solution of alkaline phosphatase conjugated anti-human IgG$_4$ monoclonal antibody prepared according to a modified procedure of M. O'Sullivan, et al, Analytical Biochem., vol. 100, page 100(1979). The monoclonal antibody is applied in a solution of 0.01 M phosphate buffered saline, pH 7.2, containing 4 wt. % polyethylene glycol having a molecular weight of 4000 (PEG 4000), 0.05 wt. % TRItON® X-405, and 0.1 wt. % sodium azide preservative. The alkaline phosphatase conjugated anti-human IgG$_4$ monoclonal antibody solution is removed from the microtiter plate well, and it is rinsed three times with the buffered rinse solution described above.

To the microtiter plate well is then added 100 microliters of a substrate solution containing $10^{-4}$ M 4 methyl umbelliferyl phosphate in 1.25 M 2-amino-2-methylpropanol, pH 9.5 in deionized water containing 0.125 mM magnesium chloride and 0.1 wt. % sodium azide. After 60 minutes, the fluorescence level is read with a fluorometer with the excitation at 365 nm and the reading at 450 nm. By comparing the reading with levels measured by repeating the procedure with control solutions having know concentrations of serum specific IgG$_4$ for timothy pollen allergen, the serum specific IgG$_4$ level in the patient serum is determined.

EXAMPLE 4

The procedure of Example 3 is repeated replacing the microtiter plate well with the timothy grass pollen extract adhered thereto with the adherent microtiter plate well products of Example 2 having other allergens adhering thereto to determine the allergen specific IgG$_4$ levels in the patient serum specific to the respective allergens.

EXAMPLE 5

A microtiter plate well product of Example 1 (to which a BSA conjugate of timothy grass pollen allergen is adhered) is rinsed for 5 minutes with a buffered rinse solution containing 0.85 wt. % sodium chloride, 0.05 wt. % TRITON® X-405, 0.01 wt. % BSA, and 0.1 wt. % sodium azide preservative in a 0.01 aqeuous phosphate buffer solution, pH 7.2, and the surplus is removed. The prerinsed microtiter plate is then contacted with patient serum containing timothy grass pollen allergen specific IgG$_4$ and incubated for 2 hours. The serum is removed, and the well washed three times with the buffered rinse solution.

The buffered rinse solution is prepared by diluting the following concentrate with 50 parts by volume distilled or deionized water to one part by volume concentrate:

| Bovine serum albumin | 0.5 wt. % |
|---|---|
| Non-ionic surfactant (TRITON® X-405) | 0.1 wt. % |
| Sodium chloride | 17 wt. % |
| Sodium azide | 2 wt. % |
| Sodium phosphate | 0.05 M |
| pH adjusted to | 7.4 wt. % |

Serum IgG$_4$ specific antibody for timothy grass pollen allergen is conjugated to the microtiter plate well surface.

The microtiter plate well is then contacted with 100 microliters of a solution of alkaline phosphatase conjugated anti-human $IgG_4$ monoclonal antibody prepared according to a modified procedure of M. O'Sullivan, et al, Analytical Biochem., vol. 100, page 100 (1979). The monoclonal antibody is applied in a solution of 0.01 M phosphate buffered saline, pH 7.2, containing 4 wt. % polyehtylene glycol having a molecular weight of 4000 (PEG 4000), 0.05 wt. % TRITON® X-405, 0.01 wt. % BSA, and 0.1 wt. % sodium azide preservative. The alkaline phosphatase conjugated anti-human $IgG_4$ monoclonal antibody solution is removed from the microtiter plate well, and it is rinsed three times with the buffered rinse solution described above.

To the microtiter plate well is then added 100 microliters of a substrate solution containing $10^{-4}$ M 4-methyl umbelliferyl phosphate in 1.25 M 2-amino-2-methylpropanol, pH 9.5 in deionized water containing 0.125 mM magnesium chloride and 0.1 wt. % sodium azide. After 60 minutes, the fluorescence level is read with a fluorometer with the excitation at 365 nm and the reading at 450 nm. By comparing the reading with levels measured by repeating the procedure with control solutions having known concentrations of serum specific $IgG_4$ for timothy pollen allergen, the serum specific $IgG_4$ level in the patient serum is determined.

EXAMPLE 6

The procedure of Example 5 is repeated replacing the microtiter plate well with the timothy grass pollen extract adhered thereto with the adherent microtiter plate well products of Example 2 inclusive, having other allergens adhering thereto to determine the allergen specific $IgG_4$ levels in the patient serum specific to the respective allergens.

EXAMPLE 7

In this example, allergen derived from Perennial Ryegrass is adhered to a well of a black, opaque polystyrene microtiter plate. The well is washed with methanol and dried. A 1:200 dilution of a 1:10 extract from Perennial Ryegrass Pollen (a 1:10 extract contains about 100,000 allergy units by the FDA suggested standards) in phosphate buffered saline having a pH of 8.5 is prepared, and 100 microliters of extract solution is pipetted into the well. After incubation for 2 hours (or overnight) at room temperature, the excess liquid is removed, and the well is washed 3 times with a 5 to 10 % aqueous solution of sucrose or sorbitol and dried.

The microtiter plate well to which perennial ryegrass pollen allergen is adhered, is washed 3 times with 0.9 % sodium chloride rinse solution containing 0.05 % TRITON® X-405 (Octylphenoxypolyethoxyethanol) Patient serum of a patient suffering an allergy to perennial ryegrass pollen (100 microliters) is added to the well and incubated at room temperature for 2 hours. The well is aspirated and washed 3 times with the above saline rinse solution.

Then 100 microliters of solution containing one microgram of alkaline phosphatase labeled monoclonal anti-$IgG_4$ antibody in a PBS solution containing 4 % (w/v) polyethylene glycol 4000 and 0.05 % TRITON® X-405 is added to the well and incubated for 2 hours at room temperature. The well is then aspirated and washed 3 times with the above rinse solution.

Then 100 microliters of an aqueous $10^{-4}$ M 4-methyl-umbelliferyl phosphate solution containing 1.25 M 2-amino-2-methyl-1-propanol buffer and 0.125 mM magnesium chloride, pH 9.5, is added to the well and incubated for 60 minutes at room temperature. The fluorescence level is read with a fluorometer with excitation at 365 nm and the reading at 450 nm.

**Claims**

1. A method for identifying and quantifying allergen specific $IgG_4$ levels in patient serum comprising:

   (a) providing an insoluble support having allergen adhering thereto, wherein the insoluble support has a plurality of reaction wells separated by opaque material;

   (b) contacting the insoluble support with patient serum for a sufficient time to permit conjugation and removing the patient serum therefrom;

   (c) contacting the insoluble support with a solution containing anti-$IgG_4$ antibody labelled with a fluorigenic enzyme for a time between 30 and 180 minutes and removing the solution containing any unconjugated anti-$IgG_4$ antibody therefrom;

   (d) contacting the insoluble support with a solution of a substrate, which undergoes reaction in the presence of the fluorigenic enzyme to yield a fluorescent product, for a time between 5 and 240 minutes;

(e) measuring the fluorescence level in the solution; and

(f) comparing the fluorescence level measured in step (e) with fluorescence levels of control solutions to determine the concentration of allergen specific $IgG_4$ in the patient serum.

2. The method of claim 1 wherein the insoluble support is a microwell plate made of opaque material.

3. The method of claim 2 wherein the microtiter plate is polystyrene or a styrene-(vinyl monomer) copolymer.

4. The method of any one of claims 1 to 3 wherein the anti-$IgG_4$ antibody is a monoclonal antibody.

5. The method of any one of claims 1 to 4 wherein the anti-$IgG_4$ antibody is labelled with alkaline phosphatase.

6. The method of claim 5 wherein the substrate is 4-methylumbelliferyl phosphate.

7. The method of any one of the preceding claims wherein the insoluble support is contacted in step (c) with anti-$IgG_4$ antibody in an aqueous solution containing from 1 to 8 wt.% polyethylene glycol having a molecular weight within the range of from 1,000 to 10,000.

8. The method of any one of the preceding claims wherein the insoluble support is contacted in step (c) with anti-$IgG_4$ antibody in an aqueous solution containing from 0.01 to 0.1 wt.% of a non-ionic surfactant.

9. The method of any one of the preceding claims wherein the allergen adhering to the insoluble support is a conjugate of allergen covalently bonded to a soluble protein or amino acid polymer.

10. The method of claim 9 wherein the allergen is a conjugate of allergen covalently bonded to a soluble protein, and the insoluble support is prerinsed with an aqueous buffered solution containing from 0.0001 to 0.5 wt.% of unconjugated animal protein of the sort to which the allergen is coupled before being contacted with patient serum.

**Patentansprüche**

1. Verfahren zur Identifizierung und Quantifizierung von allergen-spezifischen $IgG_4$-Werten in Serum von Patienten, umfassend:

(a) Bereitstellung eines unlöslichen Trägers mit hieran anhaftendem Allergen, wobei der unlösliche Träger eine Vielzahl von durch opakes Material getrennten Reaktionslöchern aufweist,

(b) Kontaktieren des unlöslichen Trägers mit Serum des Patienten für eine ausreichende Zeit zur Ermöglichung der Konjugation und Entfernen des Patientenserums hiervon,

(c) Kontaktieren des unlöslichen Trägers mit einer Lösung, die anti-$IgG_4$-Antikörper enthält, welche mit einem fluorogenen Enzym markiert sind, für eine Zeit zwischen 30 und 180 Minuten, und Entfernen der irgendwelchen unkonjugierten anti-$IgG_4$-Antikörper enthaltenden Lösung hiervon,

(d) Kontaktieren des unlöslichen Trägers mit einer Lösung eines Substrates, das in Anwesenheit des fluorogenen Enzyms eine Reaktion unter Bildung eines fluoroeszierenden Produktes erfährt, für eine Zeitdauer zwischen 5 und 240 Minuten,

(e) Messung des Fluoreszenzwertes in der Lösung, und

(f) Vergleichen der in Stufe (e) gemessenen Fluoreszenzwerte mit Fluoreszenzwerten von Kontroll-Lösungen zur Bestimmung der Konzentration von allergen-spezifischem $IgG_4$ in dem Patientenserum.

2. Verfahren nach Anspruch 1, in welchem der unlösliche Träger eine aus opakem Material hergestellte Mikro-Lochplatte ist.

3. Verfahren nach Anspruch 2, in welchem die Mikrotiterplatte Polystyrol oder ein Styrol-(Vinylmonomeres)-Copolymeres ist

**4.** Verfahren nach einem der Ansprüche 1 bis 3, in welchem der anti-IgG$_4$-Antikörper ein monoklonaler Antikörper ist.

**5.** Verfahren nach einem der Ansprüche 1 bis 4, in welchem der anti-IgG$_4$-Antikörper mit alkalischer Phosphatase markiert ist.

**6.** Verfahren nach Anspruch 5, in welchem das Substrat 4-Methylumbelliferyl-phosphat ist.

**7.** Verfahren nach einem der vorhergehenden Ansprüche, in welchem der unlösliche Träger in Stufe (c) mit anti-IgG$_4$-Antikörper in einer wässrigen Lösung, die von 1 bis 8 Gew.-% Polyethylenglykol mit einem Molekulargewicht innnerhalb des Bereiches von 1.000 bis 10.000 enthält, kontaktiert wird.

**8.** Verfahren nach einem der vorhergehenden Ansprüche, in welchem der unlösliche Träger in Stufe (c) mit anti-IgG$_4$-Antikörper in einer wässrigen Lösung, die von 0,01 bis 0,1 Gew.-% eines nicht-ionischen Tensids enthält, kontaktiert wird.

**9.** Verfahren nach einem der vorhergehenden Ansprüche, in welchem das an den unlöslichen Träger anhaftende Allergen ein Konjugat von Allergen, kovalent gebunden an ein lösliches Protein oder Aminosäurepolymeres, ist,

**10.** Verfahren nach Anspruch 9, in welchem das Allergen ein Konjugat von Allergen, kovalent gebunden an ein lösliches Protein, ist, und der unlösliche Träger mit einer wässrigen, gepufferten Lösung mit einem Gehalt von 0,0001 bis 0,5 Gew.-% an nicht-konjugiertem tierischem Protein der Sorte, an welche das Allergen vor dem Kontaktieren mit Patientenserum gekuppelt ist, vorgespült wird.

**Revendications**

**1.** Méthode d'identification et de quantification de niveaux de IgG$_4$ spécifique d'un allergène dans le sérum d'un patient consistant à :

(a) prévoir un support insoluble auquel adhère l'allergène, où le support insoluble a un certain nombre de puits de réaction qui sont séparés par un matériau opaque ;

(b) mettre le support insoluble en contact avec le sérum du patient pendant un temps suffisant pour permettre la conjugaison et l'élimination du sérum du patient ;

(c) mettre le support insoluble en contact avec une solution contenant l'anticorps anti-IgG$_4$ marqué d'une enzyme fluorigénique pendant un temps compris entre 30 et 180 minutes et en enlever la solution contenant tout anticorps anti-IgG$_4$ non conjugué ;

(d) mettre le support insoluble en contact avec une solution d'un substrat, qui subit une réaction en présence de l'enzyme fluorigénique pour donner un produit fluorescent , pendant un temps compris entre 5 et 240 minutes ;

(e) mesurer le niveau de fluorescence de la solution ; et

(f) comparer le niveau de fluorescence mesuré à l'étape (e) avec le niveau de fluorescence de solutions témoins pour déterminer la concentration de IgG$_4$ spécifique de l'allergène dans le sérum du patient.

**2.** Méthode de la revendication 1 où le support insoluble est une plaque à micropuits qui est faite en un matériau opaque.

**3.** Méthode de la revendication 2 où la plaque de microtitrage est en polystyrène ou en un copolymère de styrène-(monomère vinylique).

**4.** Méthode selon l'une quelconque des revendications 1 à 3 où l'anticorps anti-IgG$_4$ est un anticorps monoclonal.

**5.** Méthode selon l'une quelconque des revendications 1 à 4 où l'anticorps anti-IgG$_4$ est marqué par la phosphatase alcaline.

**6.** Méthode selon la revendication 5 où le substrat est le phosphate de 4-méthylumbelliféryle.

**7.** Méthode selon l'une quelconque des revendications précédentes où le support insoluble est mis en contact à l'étape (c) avec l'anticorps anti-IgG$_4$ dans une solution aqueuse contenant 1 à 8% en poids de polyéthylène glycol ayant un poids moléculaire compris entre 1.000 et 10.000.

**8.** Méthode selon l'une quelconque des revendications précédentes où le support insoluble est mis en contact à l'étape (c) avec l'anticorps anti-IgG$_4$ dans une solution aqueuse contenant 0,01 à 0,1% en poids d'un agent tensio-actif non ionique.

**9.** Méthode selon l'une quelconque des revendications précédentes où l'allergène qui adhère au support insoluble est un conjugué d'un allergène lié de manière covalente à une protéine soluble ou un polymère d'acide aminé.

**10.** Méthode selon la revendication 9 où l'allergène est un conjugué d'un allergène lié de manière covalente à une protéine soluble et le support insoluble est prérincé avec une solution aqueuse tamponnée contenant 0,0001 à 0,5% en poids de protéine animale non conjuguée du type auquel l'allergène est couplé avant mise en contact avec le sérum du patient.